# EUROPEAN PATENT SPECIFICATION

(11) **EP 3 877 396 B1**
(45) Date of publication and mention of the grant of the patent: **19.02.2025**
(21) Application number: 19801327.8
(22) Date of filing: 08.11.2019
(51) Int. Cl.: C07K 7/08, C07K 7/02, A61K 38/10

(54) **POLYPEPTIDES FOR THE TREATMENT OF CANCER**
POLYPEPTIDE ZUR BEHANDLUNG VON KREBS
POLYPEPTIDES POUR LE TRAITEMENT DU CANCER

(30) Priority: 09.11.2018 EP 18205455
(43) Date of publication of application: 15.09.2021
(73) Proprietor: Neopep Pharma GmbH & Co. KG, 30171 Hannover (DE)
(72) Inventor: FORSSMANN, Wolf-Georg, 30625 Hannover (DE)
(74) Representative: Benedum, Ulrich Max
(86) International application number: PCT/EP2019/080685
(87) International publication number: WO 2020/094843

(56) References cited:
- WO-A1-2014/198834
- WO-A1-2019/048666
- WO-A2-2008/075371
- WO-A2-2009/004054
- ONOFRIO ZIRAFI ET AL: "Proteolytic processing of human serum albumin generates EPI-X4, an endogenous antagonist of CXCR4", JOURNAL OF LEUKOCYTE BIOLOGY, vol. 99, no. 6, 1 June 2016 (2016-06-01), US, pages 863 - 868, XP055576216, ISSN: 0741-5400, DOI: 10.1189/jlb.2MR1115-521RR

## Description

The present invention concerns polypeptides and their therapeutic uses especially for the treatment of stress, immunoreaction and stroke syndromes.

### Background of the invention

When cells migrate in the body, they are guided by chemokines. Cells that are attracted by chemokines follow a signal of increasing chemokine concentration towards the source of the chemokine. There are currently four different chemokine families known which differ on the spacing of their first two cysteine residues. The so called CXC chemokines or α-chemokines comprise one amino acid a s spacer between the two cysteines, represented in the name with "X".

Accordingly, due to chemokine receptors on the surface of the cells the chemokines influence the cells. The chemokine receptors are integral membrane proteins. The CXC chemokine receptor, and especially the CXC chemokine receptor 4 (CXCR4) activates rapid growth in cancer cells and migration, forming metastases throughout the body, preferentially in the lung, bone and liver. Also, HIV can use CXCR4 to infect CD4+ T cells.

CXCR4 is involved in multiple developmental and physiological processes including stem cell homing (Mohle and Drost, 2012) and migration of immune cells (Campbell et al., 2003). Man-made CXCR4 antagonists are capable of mobilizing hematopoietic stems cells (HSCs), which are utilized for immune reconstitution after organ transplantation or chemotherapy (Ratajczak and Kim, 2012; Schroeder and DiPersio, 2012). In addition, CXCR4 is also a major co-receptor for HIV-1 entry into target cells (Feng et al., 1996; Bleul et al., 1996). Co-receptor utilization of CXCR4 is highly effective and a high proportion of CD4+ T cells express this GPCR in lymphatic tissues in vivo. Nonetheless, almost exclusively HIV-1 variants utilizing the C-C chemokine receptor type 5 (CCR5) are transmitted and found during chronic HIV-1 infection (Alkhatib et al., 1996; Deng et al., 1996; Dragic et al., 1996). It has been proposed that multiple factors contribute to the inefficient transmission of CXCR4-tropic (X4) HIV-1 strains (Margolis and Shattock, 2006). However, the mechanism(s) underlying the effective control of X4 HIV-1 in immunocompetent individuals remain poorly understood.

Research on CXCR4-antagonists has recently become an immense field of projects due to the manifold indications. In particular the efforts to find a strategy to intervene with cancer cell proliferation, differentiation, and metastasis was not so successful in clinical studies yet as expected. The development of one of the compound groups, namely AMD3100, a CXCR4-antagonists (a bicyclame compound: Hendrix and Flexner 2000), had to be stopped for long term treatments due to toxic side effects. Although AMD3100 is registered for single short applications in stem cell mobilization, it is nevertheless a challenge to find adequate antagonists to the target CXCR4.

Zirafi, O. et al. report in J. Leukoc. Biol. 99: 663-868; 2016 about an endogenous peptide inhibitor of CXCR4 as an antagonist of CXCR4. This peptide is a 16-residue fragment of human serum albumin and was isolated as an inhibitor of CXCR4-tropic human immunodeficiency virus type 1 from a blood-derived peptide library. Endogenous peptide inhibitor of CXCR4 binds the second extracellular loop of CXCR4, thereby preventing engagement of CXCL12 and antagonizing the receptor. Consequently, endogenous peptide inhibitor of CXCR4 inhibits CXCL12-mediated migration of CXCR4-expressing cells in vitro, mobilizes hematopoietic stem cells, and suppresses inflammatory responses *in vivo.* The authors discuss the generation of endogenous peptide inhibitor of CXCR4, its relevance as biomarker for disease, and its role in human immunodeficiency virus/acquired immunodeficiency syndrome pathogenesis and cancer.

WO 2009/004054 A2 discloses a peptide having the amino acid sequence Z1-LVRYTKKVPQVSTPTL-Z2 (ALB-408) and its biologically active fragments and/or variants and/or derivatives, especially amidated, acetylated, sulfated, phosphorylated and/or glycosylated derivatives, and peptides obtainable by multiple synthesis which have the biological activity of ALB408-423; wherein Z represents number of from 0 to 10 amino acid residues.

WO 2014/198834 A1 discloses peptides, in particular dimers, effective in blocking the CXC-chemokine receptor 4 (CXCR4) mediated HIV-1 NL4-3 (X4-tropic) infection with an IC50 value of less than 50µM.

It is therefore an object of the invention to provide peptides with antagonistic activities against natural CXCR4. Especially, the peptides should inhibit proliferation of cancer cells, metastasis and show the types of cancers which are addressed by the different analogs and reaction of antiinflammatory allergic reactions.

Another object of the invention is to provide a compound which is capable to reduce the receptor activity of CXCR4.

Another object of the present invention is to provide a compound which is capable to influence proliferation of a cancer cell.

Another object of the present invention is to provide a compound which is capable to influence migration or homing of a cancer cell.

A still further object of the present invention is to provide a compound which is capable to influence the formation of metastases.

Another object of the present invention is to provide a compound which is capable to treat highly aggressive tumors so that the cancer is considerably inhibited or becomes a chronic disease.

Still another object of the present invention is to provide a compound which is capable to regulate and treat various diseases, such as immune and allergic diseases, tissue growth and nervous system regulation.

### Summary of the invention

The objects of the invention are solved by any of the polypeptides of the invention. The polypeptides of the invention exhibit great therapeutic potential.

A polypeptide of the invention comprises at least one of the following amino acid sequences: LVRYTQKAPQVS, MVRYTQKAPQVS, WRYTQKAPQVS, LVRYTQCAPQVS, LVRYTQRAPQVS, LVRYTQKAPCVS, LVRYTQKAPQMS, or LVRYTQKAPQFS. The sequence LVRYTQKAPQVS is a particularly preferred embodiment.

According to the invention single or several amino acid residues in the sequence can been exchanged, deleted or added, or chemical modifications on single amino acids of said polypeptide can been introduced which result in an improved biological or pharmacological activity of the unmodified polypeptide of the invention. Respective methods for modifications are known to the skilled person.

Furthermore at least one side chain of an amino acid of said polypeptide can be chemically modified, in particular phosphorylated, amidated, acetylated, glycosylated, PEGylated, HESylated or combinations thereof.

The choice of the suitable functional group for the PEG derivative is based on the type of available reactive groups on the molecule that will be coupled to the PEG. For proteins, typical reactive amino acids include lysine, cysteine, histidine, arginine, aspartic acid, glutamic acid, serine, threonine, tyrosine. The N-terminal amino group and the C-terminal carboxylic acid can also be used as a specific site by conjugation with aldehyde functional polymers.

According to the invention the polypeptide according of the invention may comprise at least one D-amino acid. In particular, the polypeptide of the invention may be composed by a chain of D-amino acids in a retro-inverso configuration of the chain of the polypeptide of the invention.

Thus, also retro-inverso peptides of the peptides of the invention are in the scope of the present invention, as well as other derivatives stabilizing the peptide bond against peptidases. The term derivative means within the scope of the present application all length fragments including truncations at the N and C terminus, the peptide of the invention containing amino acid residue substitutions including D-amino acid residues and modified amino acid residues as well as peptides containing disulfide bonds and extension at the N and C terminus.

A further subject matter of the present invention is a medicament comprising at least one polypeptide of the invention and a pharmaceutically acceptable carrier. In one of the simplest embodiments the polypeptide of the invention can be administered in water for infusion, physiological saline, or buffered aqueous solutions. Also, other formulations are possible for example encapsulation in liposomes forming nanoparticles of various sizes, for example from 20 to 2.000 nm.

Typically, the peptide of the invention is administered in amounts of 10 - 1,000 mg/kg body weight for a time period sufficient to stop tumor growth. The time of administration is usually between two to ten weeks. The medicament of the invention is preferably suitable for oral, intravenous, intramuscular, intracutaneous, subcutaneous, intrathecal administration or present in form of an aerosol suitable for transpulmonary and intranasal administration, in particular encapsulated in liposomes; or for use in aqueous or liposomal packaging.

Subject matter of the present invention is also a polynucleotide coding for a polypeptide of the invention and/or its fragments, variants, derivatives and analogues. The polynucleotide of the invention may be constituted of DNA, RNA, genomic DNA or PNA. A polynucleotide hybridizing to a polynucleotide according to the invention that codes for a polypeptide of the invention is also subject of the present invention.

A further subject matter of the present invention is a vector containing a polynucleotide according to the invention, as well as a genetically engineered host cell containing the vector according the invention.

A still further subject matter of the present invention is an antibody directed against at least one polypeptide of the invention.

Yet another subject matter of the present invention is an antagonist/inhibitor compound directed against a polypeptide according to the invention.

Subject matter of the invention is also a peptide of the invention for use in the treatment of neurological diseases, in particular stroke, Parkinson's disease, Alzheimer's disease, multiple sclerosis; in the field of immunology in particular for the treatment of the WHIM-syndrom, lupus erythematosus and rheumatoid arthritis; in the field of oncology in particular for the treatment of cancers, in particular cancers showing the CRCX receptor such as cancer of the liver, pancreas, prostate, or breast cancer; for the treatment of lack of mobilization, proliferation and migration of stem cells, T-cell activation as well as support of immunoblasts such as CTL/PD-1; in the treatment of wounds caused by burning; for the treatment of antifibrosis; treatment or prevention of scars; for treatment of cardiologic disorders, in particular heart insufficiency; for the treatment of metabolic disorders, in particular diabetes; for the treatment of viral diseases, in particular infections with HIV-I, HIV-2, *Cytomegalo virus, Herpes simplex virus (type 1 and 2), Varicella zoster virus, Hepatitis A and Hepatitis B virus, Influenza virus, Polio virus, Rhino virus, Rubella virus, Measles virus, Rabies virus, Rous sarcoma virus, Epstein-Barr Virus;* and for the treatment of infections caused by bacteria and fungi, in particular *Pseudomonas, Candida, S. aureus;* for the treatment of infectious processes, abnormal infectious processes; treatment of growth disorders, treatment of neuronal diseases, disorders of the blood clotting cascade and hematopoiesis, vascular diseases, diseases of the immune system, and for improving wound and bone healing, pulmonary disorders, and allergies.

Another process for the manufacturing of a polypeptide according to the invention is solid-phase synthesis in terms of Merrifield synthesis or liquid-phase synthesis by methods known to the skilled person using protected amino acids, and its purification.

Still another process for the manufacturing of a polypeptide according to the invention can employ methods of heterologous expression known to the skilled person using common biotechnological vectors, and if necessary subsequent posttranslational or chemical modification.

Subject matter of the present invention is a diagnostic agent containing poly-or monoclonal antibodies according to the invention or containing the nucleic acid or mRNA coding for a polypeptide according to the invention.

A further subject matter of the invention is a diagnostic agent containing a polypeptide according to the invention or a polynucleotide according to the invention for test systems for assaying tissue, plasma, urine and cerebrospinal fluid levels of this substance, diagnostic agents and test systems detecting a polypeptide according to the invention for assaying tissue, plasma, urine and cerebrospinal fluid levels of this substance by means of mass-spectrometric methods, such as MALDI-MS or ESI-MS, in connection with sample preparation by RP-HPLC, protein precipitation and/or solid-phase extraction. Preferably methods of mass spectrometry are used for the detection of minute quantities of the molecules in the range of femto or atto molar quantities.

Also, a diagnostic agent is subject of the invention which are containing a polypeptide according to the invention as markers for viral diseases, bacterial and fungal infections, inflammatory and neoplastic processes, and as markers in inflammatory processes, disturbed inflammation reactions, tumor diseases, growth disorders, diseases of the immune system, WHIm-syndrom, lupus erythematosus and as markers in bone diseases as well as others.

The invention is further described in more detail using the peptide LVRYTQKAPQVS (SEQ ID No. 1) as a typical representative of the peptide of the invention.

### Examples

### Peptides

The peptide of SEQ ID No. 1 and various derivates thereof were synthesized by conventional solid-phase synthesis on a peptide synthesizer 9050 (Applied Biosystems) using Fmoc chemistry. The peptide was purified by RP chromatography, and its identity and purity were established by analytical RP-HPLC and MALDI-MS and LC-ESI-MS.

### Cancer cell invasion assay

The cancer cell invasion assay was performed on humanized rats (Eyol, E. et al., Oncology Reports, 28: 2177-2187, 2012). Pancreas carcinoma cells were implanted. The successful implant was observed by luminescent imaging as well as by an increase in CXCR4 expression.

After a successful implant of carcinoma cells, the rats were treated with a peptide according to SEQ ID No. 1. The results after 1 week of therapy (1 w of therapy), after 2 weeks of therapy (2 w of therapy) and 2 weeks after end of therapy are depicted in the below table.

### Result for peptide of SEQ ID No. 1

| Amount of peptide | 1 w of therapy | 2 w of therapy | 2 w after end of therapy |
|---|---|---|---|
| 0 mg (control) | Tumor growth | Tumor growth | Tumor growth |
| 10 mg | No tumor growth | Tumor growth | Tumor growth |
| 20 mg | No tumor growth | Complete Remission | Complete Remission |
| 70 mg | No tumor growth | Remission | Complete Remission |

Rats without therapy died after tumor implant within few days.

Rats with therapy using the peptide of the present invention survived over at least two weeks. The tumor growth was stopped and depending on the concentration a partial or even complete remission of the tumor was observed. No toxic effect of the peptides was observed.

### SEQUENCE LISTING

SEQ ID No. 1
   Leu Val Arg Tyr Thr Gln Lys Ala Pro Gln Val Ser
SEQ ID No. 2
   Met Val Arg Tyr Thr Gln Lys Ala Pro Gln Val Ser
SEQ ID No. 3
   Val Val Arg Tyr Thr Gln Lys Ala Pro Gln Val Ser
SEQ ID No. 4
   Leu Met Arg Tyr Thr Gln Lys Gln Pro Gln Val Ser
SEQ ID No. 5
   Leu Leu Arg Tyr Thr Gln Lys Gln Pro Gln Val Ser
SEQ ID No. 6
   Leu Val Lys Tyr Thr Gln Lys Gln Pro Gln Val Ser
SEQ ID No. 7
   Leu Val Cys Tyr Thr Gln Lys Gln Pro Gln Val Ser
SEQ ID No. 8
   Leu Val Arg Trp Thr Gln Lys Ala Pro Gln Val Ser
SEQ ID No. 9
   Leu Val Arg Tyr Ser Gln Lys Gln Pro Gln Val Ser
SEQ ID No. 10
   Leu Val Arg Tyr Thr Cys Lys Ala Pro Gln Val Ser
SEQ ID No. 11
   Leu Val Arg Tyr Thr Gln Cys Ala Pro Gln Val Ser
SEQ ID No. 12
   Leu Val Arg Tyr Thr Gln Arg Ala Pro Gln Val Ser
SEQ ID No. 13
   Leu Val Arg Tyr Thr Gln Lys Ala Pro Cys Val Ser
SEQ ID No. 14
   Leu Val Arg Tyr Thr Gln Lys Ala Pro Gln Met Ser
SEQ ID No. 15
   Leu Val Arg Tyr Thr Gln Lys Ala Pro Gln Phe Ser

## Claims

1. A polypeptide selected from the group consisting of polypeptides consisting of the amino acid sequence LVRYTQKAPQVS, MVRYTQKAPQVS, WRYTQKAPQVS, LVRYTQCAPQVS, LVRYTQRAPQVS, LVRYTQKAPCVS, LVRYTQKAPQMS, or LVRYTQKAPQFS.

2. The polypeptide according to claim 1, wherein at least one side chain of an amino acid of said polypeptide is chemically modified, in particular phosphorylated, amidated, acetylated, glycosylated, PEGylated, HESylated or combinations thereof.

3. A medicament comprising at least one polypeptide according to claims 1 or2 and a pharmaceutically acceptable carrier.

4. The medicament according to claim 3 suitable for oral, intravenous, intramuscular, intracutaneous, subcutaneous, intrathecal administration or in form of an aerosol suitable for transpulmonary administration, in particular encapsulated in liposomes; or for use in aqueous or liposomal packaging.

5. Antibodies directed against a polypeptide according to claim 1.

6. The polypeptide of claim 1 or 2 for use in the treatment of stroke, for the treatment of cancers, in particular cancers showing the CRCX receptor such as cancer of the liver, pancreas, prostate, or breast cancer; for the treatment of lack of mobilization, proliferation and migration of stem cells, T-cell activation as well as support of immunoblasts such as CTL/PD-1

7. A process for the preparation of a polypeptide according to claim 1 or 2 by extraction from hemofiltrate by cation-exchange extraction followed by elution of the adsorbed substances, renewed cation-exchange chromatography of the extract containing the peptides, and fractional reverse-phase chromatography.

8. A process for the preparation of a polypeptide according to claim 1 or 2 by solid-phase synthesis in terms of Merrifield synthesis or liquid-phase synthesis by methods known to the skilled person using protected amino acids, and its purification.

9. A process for the preparation of a polypeptide according to claim 1 or 2 by methods of heterologous expression known to the skilled person using common biotechnological vectors, and if necessary subsequent posttranslational or chemical modification.

10. A diagnostic agent containing poly-or monoclonal antibodies according to claim 5 or containing the nucleic acid or mRNA coding for a polypeptide according to claim 1.

11. A diagnostic agent containing a polypeptide according to claim 1 or 2 for test systems for assaying tissue, plasma, urine and cerebrospinal fluid levels of this substance.

12. A diagnostic agent containing a polypeptide according to claim 1 or 2 as markers for viral diseases, bacterial and fungal infections, inflammatory and neoplastic processes, and as markers in inflammatory processes, disturbed inflammation reactions, tumor diseases, growth disorders, diseases of the immune system, and as markers in bone diseases.

## Patentansprüche

1. Polypeptid, ausgewählt aus der Gruppe bestehend aus Polypeptiden mit der Aminosäuresequenz LVRYTQKAPQVS, MVRYTQKAPQVS, WRYTQKAPQVS, LVRYTQCAPQVS. LVRYTQRAPQVS, LVRYTQKAPGVS, LVRYTQKAPQMS, oder LVRYTQKAPQFS.

2. Polypeptid nach Anspruch 1, wobei mindestens eine Seitenkette einer Aminosäure des Polypeptids chemisch modifiziert ist, insbesondere phosphoryliert, amidiert, acetyliert, glycosyliert, PEGyliert, HESyliert oder Kombinationen davon,

3. Arzneimittel, umfassend mindestens ein Polypeptid nach Anspruch 1 oder 2 und einen pharmazeutisch akzeptablen Träger.

4. Arzneimittel nach Anspruch 3, geeignet zur oralen, intravenösen, intramuskulären, intrakutanen, subkutanen, intrathekalen Verabreichung oder in Form eines Aerosols, das zur transpulmonalen Verabreichung geeignet ist, insbesondere gekapselt in Liposomen; oder zur Verwendung in wässriger oder liposomaler Verpackung.

5. Antikörper, gerichtet gegen ein Polypeptid nach Anspruch 1.

6. Polypeptid nach Anspruch 1 oder 2 zur Verwendung in der Behandlung von Schlaganfall, zur Behandlung von Krebs, insbesondere von Krebsarten, die den CRCX-Rezeptor aufweisen, wie Leber-, Bauchspeicheldrüsen-, Prostata- oder Brustkrebs; zur Behandlung von Störungen der Mobilisierung, Proliferation und Migration von Stammzellen, der T-Zell-Aktivierung sowie zur Unterstützung von Immunoblasten wie CTL/PD-1.

7. Verfahren zur Herstellung eines Polypeptids nach Anspruch 1 oder 2 durch Extraktion aus Hemofiltraten mittels Kationenaustausch-Extraktion, gefolgt von der Elution der adsorbierten Substanzen, einer erneuten Kationenaustauschchromatographie des extrahierten Peptidgemischs und einer fraktionierten Reversphasen-Chromatographie.

8. Verfahren zur Herstellung eines Polypeptids nach Anspruch 1 oder 2 durch Festphasensynthese gemäß der Merrifield-Synthese oder Flüssigphasensynthese mit dem Fachmann bekannten Verfahren unter Verwendung geschützter Aminosäuren und Aufreinigung.

9. Verfahren zur Herstellung eines Polypeptids nach Anspruch 1 oder 2 durch dem Fachmann bekannte Methoden der heterologen Expression unter Verwendung üblicher biotechnologischer Vektoren und erforderlichenfalls anschließender posttranslationaler oder chemischer Modifikation.

10. Diagnostikum, enthaltend poly- oder monoklonale Antikörper nach Anspruch 5 oder enthaltend die Nukleinsäure oder mRNA, die für ein Polypeptid nach Anspruch 1 kodiert.

11. Diagnostikum, das ein Polypeptid nach Anspruch 1 oder 2 enthält, für Testsysteme zur Bestimmung des Gehalts an dieser Substanz in Gewebe, Plasma, Urin und Liquor cerebrospinalis.

12. Diagnostikum, enthaltend ein Polypeptid nach Anspruch 1 oder 2, als Marker für virale Erkrankungen, bakterielle und Pilzinfektionen, entzündliche und neoplastische Prozesse und als Marker bei Entzündungsprozessen, gestörten Entzündungsreaktionen, Tumorerkrankungen, Wachstumsstörungen, Erkrankungen des Immunsystems und als Marker bei Knochenerkrankungen.

## Revendications

1. Un polypeptide choisi dans le groupe constitué par les polypeptides composés de la séquence d'acides aminés LVRYTQKAPQVS, MVRYTQKAPQVS, WRYTQKAPQVS, LVRYTQCAPQVS. LVRYTQRAPQVS, LVRYTQKAPGVS, LVRYTQKAPQMS ou LVRYTQKAPQFS.

2. Polypeptide selon la revendication 1, dans lequel au moins une chaîne latérale d'un acide aminé dudit polypeptide est chimiquement modifiée, en particulier phosphorylée, amidée, acétylée, glycosylée, PEGylée, HESylée ou des combinaisons de celles-ci,

3. Médicament comprenant au moins un polypeptide selon les revendications 1 ou 2 et un support pharmaceutiquement acceptable.

4. Le médicament selon la revendication 3 convient pour une administration orale, intraveineuse, intramusculaire, intracutanée, sous-cutanée, intrathécale ou sous forme d'aérosol convenant à une administration transpulmonaire, en particulier encapsulé dans des liposomes ; ou pour une utilisation dans un conditionnement aqueux ou liposomal.

5. Anticorps dirigés contre un polypeptide selon la revendication 1.

6. Le polypeptide de la revendication 1 ou 2 pour le traitement des accidents vasculaires cérébraux, pour le traitement des cancers, en particulier les cancers présentant le récepteur CRCX tels que le cancer du foie, du pancréas, de la prostate ou du sein ; pour le traitement du manque de mobilisation, de prolifération et de migration des cellules souches, de l'activation des cellules T ainsi que du soutien des immunoblastes tels que CTL/PD-1.

7. Procédé de préparation d'un polypeptide selon la revendication 1 ou 2 par extraction de l'hémofiltrat par extraction par échange de cations suivie d'une élution des substances adsorbées, chromatographie par échange de cations renouvelée de l'extrait contenant les peptides, et chromatographie fractionnée en phase inverse.

8. Procédé de préparation d'un polypeptide selon la revendication 1 ou 2 par synthèse en phase solide au sens de la synthèse de Merrifield ou par synthèse en phase liquide selon les méthodes connues de l'homme du métier en utilisant des acides aminés protégés, et sa purification.

9. Procédé de préparation d'un polypeptide selon la revendication 1 ou 2 par des méthodes d'expression hétérologue connues de l'homme du métier utilisant des vecteurs biotechnologiques courants et, si nécessaire, une modification post-traductionnelle ou chimique ultérieure.

10. Agent de diagnostic contenant des anticorps poly- ou monoclonaux selon la revendication 5 ou contenant l'acide nucléique ou l'ARNm codant pour un polypeptide selon la revendication 1.

11. Agent de diagnostic contenant un polypeptide selon la revendication 1 ou 2 pour des systèmes d'essai permettant de doser les niveaux de cette substance dans les tissus, le plasma, l'urine et le liquide céphalo-rachidien.

12. Agent de diagnostic contenant un polypeptide selon la revendication 1 ou 2 comme marqueur de maladies virales, d'infections bactériennes et fongiques, de processus inflammatoires et néoplasiques, et comme marqueur dans les processus inflammatoires, les réactions inflammatoires perturbées, les maladies tumorales, les troubles de la croissance, les maladies du système immunitaire, et comme marqueur dans les maladies osseuses.
